# EUROPÄISCHE PATENTSCHRIFT

(11) **EP 3 274 328 B1**
(45) Veröffentlichungstag und Bekanntmachung des Hinweises auf die Patenterteilung: **28.08.2019**
(21) Anmeldenummer: 16711295.2
(22) Anmeldetag: 23.03.2016
(51) Int. Cl.: C07C 215/50, C08G 59/50, C09D 163/00, C08L 63/00

(54) **AMIN FÜR SCHNELL HÄRTENDE EPOXIDHARZ-ZUSAMMENSETZUNGEN**
AMINE FOR FAST SETTING EPOXY RESIN COMPOSITIONS
AMINE POUR COMPOSITIONS DE RÉSINE ÉPOXY À DURCISSEMENT RAPIDE

(30) Priorität: 23.03.2015 EP 15160411
(43) Veröffentlichungstag der Anmeldung: 31.01.2018
(73) Patentinhaber: Sika Technology AG, 6340 Baar (CH)
(72) Erfinder: KASEMI, Edis, 8046 Zürich (CH); KRAMER, Andreas, 8006 Zürich (CH); STADELMANN, Ursula, 8046 Zürich (CH); BURCKHARDT, Urs, 8049 Zürich (CH)
(74) Vertreter: Sika Patent Attorneys
(86) Internationale Anmeldenummer: PCT/EP2016/056361
(87) Internationale Veröffentlichungsnummer: WO 2016/151006

(56) Entgegenhaltungen:
- WO-A1-2013/010841
- CN-A- 103 102 506
- US-A- 4 399 268
- International Union Of Pure And Applied Chemistry: "IUPAC Gold Book - alkyl groups", , 2008, XP055494035, Retrieved from the Internet: URL:https://goldbook.iupac.org/html/A/A002 28.html [retrieved on 2018-07-20]

## Beschreibung

### Technisches Gebiet

Die Erfindung betrifft das Gebiet der Amine, Härter für Epoxidharze, Epoxidharz-Zusammensetzungen, sowie deren Verwendung, insbesondere als Beschichtung, Belag oder Anstrich.

### Stand der Technik

In Epoxidharz-Produkten, die auch bei verhältnismässig tiefen Umgebungstemperaturen, etwa im Bereich von 0 bis 10 °C, rasch aushärten sollen, werden als Härter oft sogenannte Mannich-Basen eingesetzt. Mannich-Basen stellen Kondensationsprodukte aus Phenolen, Aldehyden und Polyaminen dar, welche zu einer schnellen Reaktion mit Epoxidharzen fähig sind, wodurch Epoxidharz-Produkte auch bei niedrigen Umgebungstemperaturen schnell Festigkeit aufbauen und klebfrei werden und somit schon bald nach der Applikation belastbar sind.

Mannich-Basen nach dem Stand der Technik enthalten typischerweise einen beträchtlichen Anteil (bis gegen 50 Gewichts-%) an Phenolen, insbesondere Phenol oder Alkylphenole wie Kresol, tert.Butylphenol oder Nonylphenol. Solche Phenole sind toxische Substanzen, die bei der Aushärtungsreaktion nicht ins Epoxidharz eingebaut werden. Aufgrund einer stärkeren Gewichtung von EHS-Aspekten durch Berufsverbände und Verbraucher sowie schärferer staatlicher Regulierung sind Phenol-haltige Härter unter Druck geraten. Es wurden deshalb Wege gesucht, um Phenol-freie Mannich-Basen herzustellen. Die Entfernung der Phenole aus herkömmlichen Mannich-Basen über physikalische Prozesse ist aber aufwändig und kommerziell kaum interessant. Die Reduktion des Phenol-Gehalts über die chemische Prozessführung, insbesondere durch ein niedrigeres Phenol-Polyamin-Verhältnis, führt typischerweise zu hochviskosen, dunkelfarbigen Mannich-Basen mit hohen Anteilen an Polyamin-verbrückten Mehrkernverbindungen. Solche Härter können nur in Farbton-unkritischen Anwendungen, beispielsweise Grundierungen, eingesetzt werden und müssen zur Verarbeitung verdünnt werden, entweder mittels (VOC-)Lösemitteln bzw. Verdünnern, was weitere EHS-Probleme verursacht, oder mittels niedrigviskosen Polyaminen, was meist zu Blushing-Effekten führt. Als "Blushing-Effekte" werden bei der Aushärtung auftretende Oberflächenmängel wie Trübungen, Flecken, Rauheit und Klebrigkeit bezeichnet, welche durch Salzbildung ("Blushing") von Aminen mit Kohlendioxid (CO₂) aus der Luft verursacht werden und besonders bei hoher Luftfeuchtigkeit und tiefen Temperaturen auftreten.

US 4,399,268 beschreibt Phenolgruppen-haltige Härter für Epoxidharze mit hoher Chemikalienbeständigkeit. Die offenbarten Härter werden durch reduktive Alkylierung von Polyaminen mit phenolgruppen-haltigen Aldehyden hergestellt und sind Phenol-frei. Allerdings sind sie recht hochviskos und führen zu einer verstärkten Vergilbung.

WO 2013/010841 beschreibt ebenfalls Phenolgruppen-haltige Härter für Epoxidharze aus reduktiver Alkylierung. Die offenbarten Härter sind Phenol-frei und weisen eine niedrige Viskosität auf, sind aber bezüglich Aushärtungsgeschwindigkeit und Viskosität noch verbesserungsfähig.

### Darstellung der Erfindung

Aufgabe der vorliegenden Erfindung ist es daher, einen Härter mit hoher Reaktivität gegenüber Epoxidharzen zur Verfügung zu stellen, welcher Phenol-frei und niedrigviskos ist, mit Epoxidharzen schnell reagiert, auch bei tiefen Umgebungstemperaturen, nicht zu Blushing-Effekten führt, hellfarbig ist und keine verstärkte Vergilbung verursacht.

Überraschenderweise wird diese Aufgabe mit dem Amin der Formel (I) wie in Anspruch 1 beschrieben gelöst. Das Amin der Formel (I) ist in einem einfachen Verfahren aus gut erhältlichen Ausgangsstoffen Phenol-frei herstellbar und ist überraschend niedrigviskos. Es reagiert mit Epoxidharzen mit hoher Geschwindigkeit, sowohl bei Raumtemperatur als auch bei niedrigeren Temperaturen, ohne dass dabei Blushing-Effekte auftreten, und ermöglicht daher auch bei ungünstigen Anwendungsbedingungen einen raschen Festigkeitsaufbau und eine hohe Produktqualität. Besonders überraschend ist die Tatsache, dass das Amin der Formel (I) fast farblos herstellbar ist und zu einer aussergewöhnlich geringen Vergilbung von Epoxidharz-Produkten nach der Aushärtung führt. Das Amin verdünnt Epoxidharze überraschend gut, besser als ähnliche Amine aus dem Stand der Technik. Als Härter für Epoxidharze ermöglicht es einen sehr raschen Festigkeitsaufbau, auch bei verhältnismässig tiefen Temperaturen wie beispielsweise bei 8 °C, wobei qualitativ hochstehende Kunsttoffe von hoher Härte und ebenmässiger, nichtklebriger Oberfläche mit hohem Glanz erhalten werden. Dank seiner Hellfarbigkeit aus der erfindungsgemässen Herstellung und der geringen Vergilbung kann es ohne Einschränkung auch in farblich anspruchsvollen Produkten wie Deckbeschichtungen oder Bodenbelägen eingesetzt werden. Im Vergleich mit aus dem Stand der Technik bekannten ähnlichen Aminen ermöglicht das Amin der Formel (I) Epoxidharz-Zusammensetzungen mit einer besonders attraktiven Kombination aus niedriger Viskosität und schnellem Festigkeitsaufbau, wie sie aufgrund des Standes der Technik nicht zu erwarten war.

Dank seiner niedrigen Viskosität kann das Amin der Formel (I) auch ohne den Einsatz von Verdünnern verwendet werden und ermöglicht somit die Formulierung von emissionsarmen Epoxidharz-Produkten, insbesondere Epoxidharz-Beschichtungen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Amin der Formel (I), 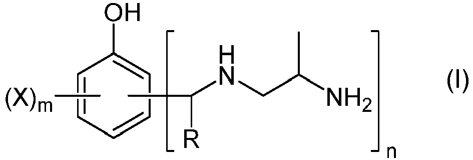 wobei
m für 0 oder 1 oder 2 steht,
n für 1 oder 2 oder 3 steht,
R für einen Wasserstoff-Rest oder Alkyl-Rest mit 1 bis 8 C-Atomen oder Phenyl-Rest steht und
X für gleiche oder verschiedene Reste ausgewählt aus der Gruppe bestehend aus Hydroxyl und Alkyl, Alkenyl und Alkoxy mit jeweils 1 bis 18 C-Atomen, welche gegebenenfalls Ether-Sauerstoff, Hydroxyl-Sauerstoff oder Amin-Stickstoff enthalten, oder für einen N-Pyrrolidinylmethyl- oder N-Piperidinylmethyl- oder N-Morpholinylmethyl-Rest steht.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.

Als "primäre Aminogruppe" wird eine NH₂-Gruppe bezeichnet, die an einen organischen Rest gebunden ist und als "sekundäre Aminogruppe" wird eine NH-Gruppe bezeichnet, die an zwei organische Reste, welche auch gemeinsam Teil eines Rings sein können, gebunden ist.

Als "Aminwasserstoff" werden die Wasserstoffatome von primären und sekundären Aminogruppen bezeichnet.

Als "Aminwasserstoff-Equivalentgewicht" wird der Gewichtsanteil eines Härters oder eines Amins pro im Härter oder im Amin vorhandenem Aminwasserstoff bezeichnet.

Als "Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.

Als "Viskosität" wird die dynamische Viskosität oder Scherviskosität bezeichnet, welche durch das Verhältnis zwischen der Schubspannung und der Scherrate (Geschwindigkeitsgefälle) definiert ist und wie in den Ausführungsbeispielen beschrieben bestimmt wird.

Als "Molekulargewicht" wird die molare Masse (in Gramm pro Mol) eines Moleküls bezeichnet. Als "mittleres Molekulargewicht" wird das Zahlenmittel Mₙ einer oligomeren oder polymeren Mischung von Molekülen bezeichnet, welches üblicherweise mittels Gelpermeationschromatographie (GPC) gegen Polystyrol als Standard bestimmt wird.

Als "Raumtemperatur" wird eine Temperatur von 23 °C bezeichnet.

Als "Phenol-frei" wird eine Substanz oder Zusammensetzung bezeichnet, die frei ist von Phenol oder Alkylphenolen wie beispielsweise Kresol, tert.Butylphenol oder Nonylphenol, oder höchstens Spuren solcher Phenole in einer Konzentration unterhalb von 0.1 Gewichts-% enthält.

Bevorzugt steht X für gleiche oder verschiedene Reste ausgewählt aus der Gruppe bestehend aus Hydroxyl und Alkyl, Alkenyl und Alkoxy mit jeweils 1 bis 15 C-Atomen, welche gegebenenfalls Ether-Sauerstoff, Hydroxyl-Sauerstoff oder Amin-Stickstoff enthalten.

Besonders bevorzugt steht X für Hydroxy, Methyl, Methoxy, tert.Butyl, Nonyl, Dodecyl, Pentadeca-8,11,14-trienyl, N,N-Dimethylaminomethyl, N,N-Diethylaminomethyl, N-Methyl-N-ethylaminomethyl, N-Methyl-N-butylaminomethyl, N,N-Bis(hydroxyethyl)aminomethyl, N-Methyl-N-hydroxyethylaminomethyl, N-Ethyl-N-hydroxyethylaminomethyl, N-Butyl-N-hydroxyethylaminomethyl, N-Pyrrolidinylmethyl, N-Piperidinylmethyl, N-Morpholinylmethyl, (3-(N,N-Dimethylamino)propyl)aminomethyl oder (3-(3-(N,N-Dimethylamino)propyl)aminopropyl)aminomethyl, insbesondere für Hydroxy, Methoxy oder N,N-Dimethylaminomethyl.

Bevorzugt steht R für einen Wasserstoff-Rest oder für einen Alkyl-Rest mit 1 bis 4 C-Atomen, insbesondere Methyl, Ethyl, Isopropyl oder 3-Heptyl, oder für einen Phenyl-Rest.

Besonders bevorzugt steht R für einen Wasserstoff-Rest oder für einen Methyl-Rest, insbesondere für einen Wasserstoff-Rest.

Die bevorzugten Amine der Formel (I) zeichnen sich durch eine besonders gute Herstellbarkeit, eine besonders niedrige Viskosität und besonders gute Eigenschaften für die beschriebenen Verwendungen aus.

Besonders bevorzugt stehen m für 0 oder1, n für 1 und R für einen Wasserstoff- oder Methyl-Rest. Dabei steht X insbesondere für Hydroxy oder Methoxy. Ein solches Amin der Formel (I) ist besonders rein herstellbar, besonders niedrigviskos und besonders reaktiv.

Weiterhin besonders bevorzugt stehen (m+n) für 3, R für einen Wasserstoff-Rest und X für einen N,N-Dialkyl-Rest, welcher gegebenenfalls eine oder zwei Hydroxylgruppen enthält, oder für einen N-Alkyl-Rest, welcher gegebenenfalls eine Hydroxylgruppe oder ein oder zwei Amin-Stickstoffe enthält, oder für einen N-Pyrrolidinylmethyl- oder N-Piperidinylmethyl- oder N-Morpholinylmethyl-Rest.

Insbesondere stehen (m+n) für 3, R für einen Wasserstoff-Rest und X für N,N-Dimethylaminomethyl.

Ein solches Amin der Formel (I) ist besonders einfach herstellbar und niedrigviskos.

Ganz besonders bevorzugt ist das Amin der Formel (I) ausgewählt aus der Gruppe bestehend aus 2-(((2-Aminopropyl)amino)methyl)phenol, 2-(1-((2-Aminopropyl)amino)ethyl)phenol, 2-(((2-Aminopropyl)amino)methyl)-4,6-bis(dimethylaminomethyl)phenol, 4-(((2-Aminopropyl)amino)methyl)-2,6-bis(dimethylaminomethyl)phenol, 2,4-Bis(((2-aminopropyl)amino)methyl)-6-dimethylaminomethylphenol, 2,6-Bis(((2-aminopropyl)amino)methyl)-4-dimethylaminomethylphenol und 2,4,6-Tris-(((2-aminopropyl)amino)methyl)phenol.

Dabei zeichnen sich 2-(((2-Aminopropyl)amino)methyl)phenol und 2-(1-((2-Aminopropyl)amino)ethyl)phenol durch eine besonders hohe Reaktivität, eine besonders niedrige Viskosität und besonders wenig Neigung zu Blushing-Effekten aus, was für die Verwendung in Epoxidharz-Beschichtungen besonders vorteilhaft ist.

2-(((2-Aminopropyl)amino)methyl)-4,6-bis(dimethylaminomethyl)phenol, 4-(((2-Aminopropyl)amino)methyl)-2,6-bis(dimethylaminomethyl)phenol, 2,4-Bis(((2-aminopropyl)amino)methyl)-6-dimethylaminomethylphenol und 2,6-Bis(((2-aminopropyl)amino)methyl)-4-dimethylaminomethylphenol zeichnen sich durch eine besonders einfache Herstellbarkeit und eine besonders hohe Reaktivität mit Epoxidharzen aus.

2,4,6-Tris-(((2-aminopropyl)amino)methyl)phenol zeichnet sich durch eine besonders einfache Herstellbarkeit und eine besonders hohe Funktionalität in Bezug auf Aminwasserstoffe aus.

Ein weiterer Gegenstand der Erfindung ist ein Verfahren zur Herstellung des Amins der Formel (I), wobei 1,2-Propylendiamin
- entweder unter reduktiver Alkylierung mit mindestens einem Aldehyd oder Keton der Formel (II) und Wasserstoff 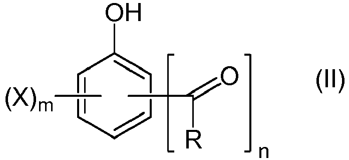
- oder unter Umaminierung mit einer Mannich-Base der Formel (III) 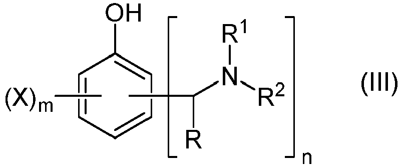 umgesetzt wird, wobei
   R¹ und R² jeweils für gleiche oder verschiedene Alkyl-, Cycloalkyl-, oder Aralkyl-Reste mit 1 bis 4 C-Atomen, welche gegebenenfalls Ether-Sauerstoff oder Amin-Stickstoff enthalten, oder zusammen für einen Alkylen-Rest mit 4 bis 8 C-Atomen, welcher gegebenenfalls Ether-Sauerstoff oder Amin-Stickstoff enthält, stehen,
   und m, n, X und R die bereits genannten Bedeutungen aufweisen.

Aus diesem Verfahren erhaltene Reaktionsprodukte sind Phenol-frei, weisen einen hohen Gehalt an Aminen der Formel (I) auf und sind besonders geeignet zur Verwendung als Härter für Epoxidharze.

Das Reaktionsprodukt enthaltend mindestens ein Amin der Formel (I) aus dem beschriebenen Verfahren kann ohne weitere Aufarbeitung oder Reinigung als Härter für Epoxidharze verwendet werden, oder es kann ein Reinigungsschritt durchgeführt werden, bevor es weiterverwendet wird.

In Formel (II) steht n bevorzugt für 1.

In Formel (III) steht (m+n) bevorzugt für 3.

R¹ und R² stehen bevorzugt unabhängig voneinander jeweils für Methyl, Ethyl, Isopropyl, Butyl oder Isobutyl, oder zusammen für 1,4-Butylen oder 1,5-Pentylen oder 3-Oxa-1,5-pentylen.

Besonders bevorzugt stehen R¹ und R² jeweils für Methyl.

Die Verfahrensvariante der reduktiven Alkylierung von 1,2-Propylendiamin mit dem Aldehyd oder Keton der Formel (II) kann direkt mit molekularem Wasserstoff oder indirekt durch Wasserstoff-Transfer von anderen Reagentien erfolgen. Bevorzugt wird molekularer Wasserstoff verwendet. Die Reaktionsbedingungen werden vorteilhaft so gewählt, dass nur eine der beiden Aminogruppen von 1,2-Propylendiamin mit guter Selektivität einfach alkyliert wird und der aromatische Ring nicht hydriert wird. Bevorzugt wird bei einem Wasserstoff-Druck von 5 bis 100 bar, einer Temperatur von 40 bis 120 °C und in Anwesenheit eines geeigneten Katalysators gearbeitet. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel. Die Herstellung des Amins der Formel (I) durch reduktive Alkylierung ist für die Verwendung als Härter für Epoxidharze besonders vorteilhaft, da primäre Aminogruppen mit guter Selektivität einfach alkyliert werden, während sekundäre Aminogruppen kaum weiter alkyliert werden. Das Reaktionsprodukt kann deshalb ohne weitere Aufarbeitung oder Reinigung als Härter für Epoxidharze verwendet werden.

In einer Ausführungsform der reduktiven Alkylierung wird 1,2-Propylendiamin gegenüber dem Aldehyd oder Keton der Formel (II) in einem Molverhältnis von ca. 1/1 eingesetzt. Dabei wird 1,2-Propylendiamin bevorzugt in einem Lösemittel gelöst, welches nach der Umsetzung destillativ entfernt wird. Diese Herstellung ist besonders ökonomisch. Ein auf diese Weise hergestelltes Amin der Formel (I) weist gewisse Anteile an weiteren Alkylierungsprodukten auf, wie sie in den folgenden Formeln für den Fall, dass der Index n für 1 steht, beispielhaft dargestellt sind:

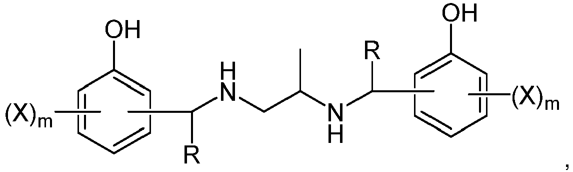

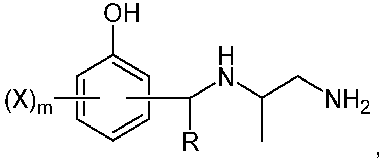

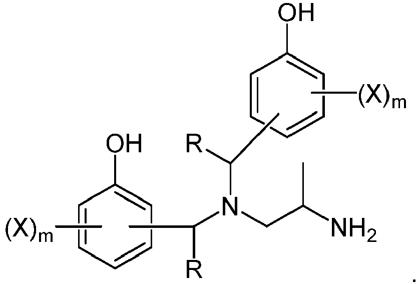

Bevorzugt wird 1,2-Propylendiamin gegenüber dem Aldehyd oder Keton der Formel (II) im stöchiometrischen Überschuss eingesetzt und der Überschuss vor oder bevorzugt nach der Reduktion entfernt, insbesondere durch Destillation.

Bevorzugt wird die reduktive Alkylierung mit einem Verhältnis zwischen der Anzahl 1,2-Propylendiamin-Moleküle und der Anzahl Aldehyd- oder Keton-Gruppen des Aldehyds oder Ketons der Formel (II) von mindestens 1.5/1, besonders bevorzugt mindestens 2/1, durchgeführt. Nach Entfernung von 1,2-Propylendiamin enthält ein solches Reaktionsprodukt einen besonders hohen Anteil an Amin der Formel (I).

Als Aldehyd oder Keton der Formel (II) geeignet sind insbesondere 2-Hydroxybenzaldehyd (Salicylaldehyd), 3-Hydroxybenzaldehyd, 4-Hydroxybenzaldehyd, 2-Hydroxy-3-methylbenzaldehyd, 2-Hydroxy-5-methylbenzaldehyd, 4-Hydroxy-3,5-dimethylbenzaldehyd, 2-Hydroxy-3-methoxybenzaldehyd (o-Vanillin), 2-Hydroxy-4-methoxybenzaldehyd, 2-Hydroxy-5-methoxybenzaldehyd, 3-Hydroxy-4-methoxybenzaldehyd (Isovanillin), 4-Hydroxy-2-methoxybenzaldehyd, 4-Hydroxy-3-methoxybenzaldehyd (Vanillin), 6-Hydroxy-2,4-dimethoxybenzaldehyd, 2,3-Dihydroxybenzaldehyd, 2,4-Dihydroxybenzaldehyd (β-Resorcinaldehyd), 2,5-Dihydroxybenzaldehyd (Gentisinaldehyd), 3,4-Dihydroxybenzaldehyd, 3,5-Dihydroxybenzaldehyd, 2,3,4-Trihydroxybenzaldehyd, 2'-Hydroxyacetophenon, 3'-Hydroxyacetophenon, 4'-Hydroxyacetophenon, 4'-Hydroxy-3'-methoxyacetophenon (Acetovanillon), 4'-Hydroxy-3',5'-dimethoxyacetophenon, 2',4'-Dihydroxyacetophenon (Resacetophenon), 2',5'-Dihydroxyacetophenon (Chinacetophenon), 2',6'-Dihydroxyacetophenon (2-Acetylresorcin), 2',4'-Dihydroxyacetophenon, 4-Hydroxybenzophenon oder 2-Hydroxy-4-methoxy-benzophenon.

Als Aldehyd oder Keton der Formel (II) bevorzugt sind Salicylaldehyd, 3-Hydroxybenzaldehyd, 4-Hydroxybenzaldehyd, o-Vanillin, Isovanillin, Vanillin, β-Resorcinaldehyd, Gentisinaldehyd, 3,4-Dihydroxybenzaldehyd, 2'-Hydroxyacetophenon, 3'-Hydroxyacetophenon, 4'-Hydroxyacetophenon, Acetovanillon oder Resacetophenon, insbesondere Salicylaldehyd oder 2'-Hydroxyacetophenon.

Bevorzugt wird die reduktive Alkylierung mit Salicylaldehyd oder 2'-Hydroxyacetophenon als Aldehyd oder Keton der Formel (II) durchgeführt.
Ein Amin der Formel (I) aus diesem Verfahren weist eine hohe Reinheit auf und zeichnet sich durch eine besonders niedrige Viskosität, eine geringe Eigenfärbung und eine hohe Reaktivität aus. Bevorzugt stehen dabei m für 0 oder 1, n für 1 und R für einen Wasserstoff- oder Methyl-Rest.

Bei der Verfahrensvariante der Umaminierung einer Mannich-Base der Formel (III) mit 1,2-Propylendiamin wird ein Amin R¹-NH-R² freigesetzt. Dieses wird bevorzugt aus der Reaktionsmischung entfernt, insbesondere mittels Destillation.

Bevorzugt weist das freigesetzte Amin R¹-NH-R² einen niedrigeren Siedepunkt auf als 1,2-Propylendiamin. In diesem Fall wird die Reaktionsmischung während der Umsetzung bevorzugt bei Normaldruck am Rückfluss erwärmt, wobei das Kühlwasser eine Temperatur aufweist, bei welcher 1,2-Propylendiamin kondensiert, nicht aber das freigesetzte Amin R¹-NH-R². Bevorzugt wird bei einer Temperatur von 60 bis 140 °C gearbeitet.

Die Reaktionsbedingungen der Umaminierung werden vorteilhaft so gewählt, dass nur eine der beiden Aminogruppen von 1,2-Propylendiamin alkyliert wird und somit möglichst wenig Mehrkernverbindungen entstehen.

Bevorzugt wird die Umaminierung mit einem Verhältnis zwischen der Anzahl 1,2-Propylendiamin-Molekülen und der Anzahl Aminoalkyl-Substituenten der Mannich-Base der Formel (III) von mindestens 1/1, insbesondere mindestens 1.5/1, besonders bevorzugt mindestens 2/1, durchgeführt.

In einer bevorzugten Ausführungsform der Umaminierung wird 1,2-Propylendiamin stöchiometrisch gegenüber der Anzahl Aminoalkyl-Substituenten der Mannich-Base eingesetzt und das freigesetzte Amin R¹-NH-R² während oder nach der Umsetzung aus der Reaktionsmischung entfernt, insbesondere mittels Destillation. Diese Herstellung ist besonders ökonomisch.

In einer besonders bevorzugten Ausführungsform der Umaminierung wird 1,2-Propylendiamin im stöchiometrischen Überschuss gegenüber der Anzahl Aminoalkyl-Substituenten der Mannich-Base eingesetzt und der Überschuss nach der Reduktion entfernt, insbesondere mittels Destillation. Dabei beträgt das Verhältnis zwischen der Anzahl 1,2-Propylendiamin-Molekülen und der Anzahl Aminoalkyl-Substituenten der Mannich-Base bevorzugt mindestens mindestens 1.5/1, besonders bevorzugt mindestens 2/1. Das so erhaltene Reaktionsprodukt weist einen besonders hohen Anteil an Amin der Formel (I) und einen besonders geringen Anteil an mehrkernigen Nebenprodukten auf.

Als Mannich-Base der Formel (III) geeignet sind insbesondere Umsetzungsprodukte von mindestens einem Phenol mit mindestens einem Aldehyd und mindestens einem sekundären Amin nach bekannten Verfahren.

Als Phenol geeignet ist insbesondere Phenol, o-Kresol, m-Kresol, p-Kresol, 4-tert.Butylphenol, 4-Nonylphenol, 4-Dodecylphenol, 3-(Pentadeca-8,11,14-trienyl)phenol (Cardanol), 2,3-Dimethylphenol (o-Xylenol), 2,4-Dimethylphenol (m-Xylenol), 2,5-Dimethylphenol (p-Xylenol), 2,6-Dimethylphenol, 3,4-Dimethylphenol, 3,5-Dimethylphenol, 2-Methoxyphenol (Guajacol), 3-Methoxyphenol, 4-Methoxyphenol, 2,6-Dimethoxyphenol, Brenzkatechin, Resorcin, Hydrochinon oder Pyrogallol. Bevorzugt ist Phenol, o-Kresol, m-Kresol, p-Kresol, 4-tert.Butylphenol, 4-Nonylphenol, 4-Dodecylphenol, Cardanol, o-Xylenol, m-Xylenol, p-Xylenol, Guajacol, 3-Methoxyphenol, 4-Methoxyphenol, Resorcin oder Hydrochinon. Besonders bevorzugt ist Phenol, o-Kresol, m-Kresol, p-Kresol, Cardanol oder Resorcin, insbesondere Phenol oder Cardanol.

Als Aldehyd geeignet ist insbesondere Formaldehyd, Acetaldehyd, Propionaldehyd, Isobutyraldehyd, 2-Ethylhexanal oder Benzaldehyd. Bevorzugt ist Formaldehyd.

Als sekundäres Amin geeignet ist insbesondere Dimethylamin, Diethylamin, Diisopropylamin, Dibutylamin, Diisobutylamin, Methylethylamin, Methylbutylamin, Pyrrolidin, Piperidin, Morpholin. Bevorzugt ist Dimethylamin.

Als Mannich-Base der Formel (III) besonders bevorzugt ist 2,4,6-Tris(N,N-dimethylaminomethyl)phenol. Diese Mannich-Base ist besonders gut und aus günstigen Rohstoffen herstellbar, kommerziell in guter Qualität (hohe Reinheit, Phenol-frei) verfügbar und besonders geeignet für Umaminierungsreaktionen, da das daraus freigesetzte Dimethylamin einen tiefen Siedepunkt aufweist und dadurch in Anwesenheit von 1,2-Propylendiamin selektiv aus der Reaktionsmischung entfernt werden kann.

Bevorzugt wird die Umaminierung mit 2,4,6-Tris(N,N-dimethylaminomethyl)-phenol als Mannich-Base der Formel (III) durchgeführt.

Ein Amin der Formel (I) aus diesem Verfahren zeichnet sich durch eine hohe Reinheit, eine hohe Funktionalität in Bezug auf Aminwasserstoffe, eine niedrige Viskosität und eine geringe Eigenfärbung aus.

Bevorzugt stehen dabei (m+n) für 3, R für einen Wasserstoff-Rest und X insbesondere für N,N-Dimethylaminomethyl.

Das Verfahren unter Umaminierung mit 2,4,6-Tris(N,N-dimethylaminomethyl)-phenol kann so durchgeführt werden, dass nicht alle drei Aminoalkylsubstituenten ausgetauscht werden, wobei insbesondere Produkte wie in den folgenden Formeln beispielhaft dargestellt erhalten werden:

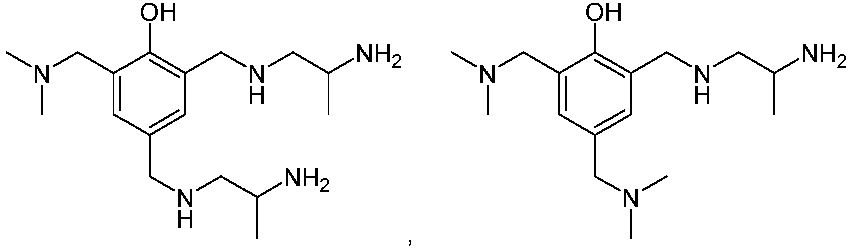

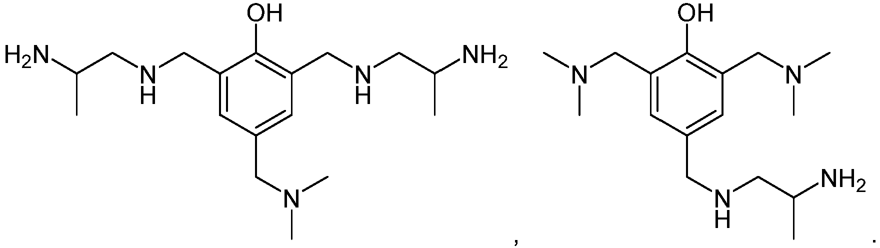

Diese Produkte stellen Amine der Formel (I) dar, bei welchen X für Dimethylaminomethyl und (m+n) für 3 steht.

Ein Reaktionsprodukt aus der Umaminierung mit 2,4,6-Tris(N,N-dimethylaminomethyl)phenol enthält typischerweise eine Mischung aus solchen Aminen der Formel (I) und dem vollständig umaminierten Produkt 2,4,6-Tris-(((2-aminopropyl)amino)methyl)phenol, wobei der Anteil an nur teilweise umaminierten Produkten mittels der Stöchiometrie, der Dauer der Umsetzung und/oder der Art der Entfernung des freigesetzten Dimethylamins gesteuert werden kann. Ebenfalls enthalten sind typischerweise Anteile von mehrkernigen Verbindungen.

Das Amin der Formel (I) ist besonders geeignet als Härter für Epoxidharze. Es weist eine für Phenolgruppen-haltige Amine niedrige Viskosität auf und ermöglicht niedrigviskose, gut verarbeitbare Epoxidharz-Produkte, welche auch bei verhältnismässig tiefen Umgebungstemperaturen schnell aushärten und dabei eine hohe Härte und eine schöne, d.h. ebenmässige und nichtklebrige, Oberfläche ausbilden und kaum vergilben.

Ein weiterer Gegenstand der Erfindung ist somit die Verwendung von mindestens einem Amin der Formel (I) als Härter für Epoxidharze.

Ein bevorzugter Härter für Epoxidharze enthält zusätzlich zum Amin der Formel (I) weitere Bestandteile, insbesondere weitere Amine und/oder Additive. Ein weiterer Gegenstand der Erfindung ist somit ein Härter für Epoxidharze, enthaltend mindestens ein Amin der Formel (I) und mindestens ein weiteres Amin und/oder mindestens ein Additiv. Das weitere Amin ist dabei kein Amin der Formel (I).

Als weiteres Amin geeignet sind insbesondere Polyamine, welche mindestens zwei, insbesondere mindestens drei, gegenüber Epoxidgruppen reaktive Aminwasserstoffe aufweisen, insbesondere die folgenden Polyamine:
- aliphatische, cycloaliphatische oder arylaliphatische primäre Diamine, insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, Bis(4-aminocyclohexyl)methan (H₁₂-MDA), Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (Isophorondiamin oder IPDA), 2- oder 4-Methyl-1,3-diaminocyclohexan oder Mischungen davon, 1,3-Bis(aminomethyl)cyclohexan, 1,4-Bis(aminomethyl)cyclohexan, 2,5(2,6)-Bis(aminomethyl)bicyclo[2.2.1]heptan (NBDA), 3(4),8(9)-Bis(aminomethyl)tricyclo-[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA) oder 1,4-Bis(aminomethyl)benzol;
- aliphatische, cycloaliphatische oder arylaliphatische primäre Triamine, insbesondere 4-Aminomethyl-1,8-octandiamin, 1,3,5-Tris(aminomethyl)benzol, 1,3,5-Tris(aminomethyl)cyclohexan, Tris(2-aminoethyl)amin, Tris(2-amino-propyl)amin oder Tris(3-aminopropyl)amin;
- Ethergruppen-haltige aliphatische primäre Di- oder Triamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder höhere Oligomere dieser Diamine, Bis(3-aminopropyl)polytetrahydrofurane oder andere Polytetrahydrofurandiamine, cycloaliphatische Ethergruppen-haltige Diamine aus der Propoxylierung und nachfolgenden Aminierung von 1,4-Dimethylolcyclohexan, erhältlich insbesondere als Jeffamine® RFD-270 (von Huntsman), oder Polyoxyalkylendi- oder -triamine, welche typischerweise Produkte aus der Aminierung von Polyoxyalkylendi- oder -triolen darstellen und beispielsweise erhältlich sind unter dem Namen Jeffamine® (von Huntsman), unter dem Namen Polyetheramine (von BASF) oder unter dem Namen PC Amine® (von Nitroil). Insbesondere geeignete Polyoxyalkylendi- oder -triamine sind Jeffamine® D-230, Jeffamine® D-400, Jeffamine® D-2000, Jeffamine® EDR-104, Jeffamine® EDR-148, Jeffamine® EDR-176, Jeffamine® T-403, Jeffamine® T-3000, Jeffamine® T-5000, oder entsprechende Amine von BASF oder Nitroil;
- sekundäre Aminogruppen aufweisende Polyamine mit zwei primären aliphatischen Aminogruppen, wie insbesondere 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA) oder höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin oder N,N'-Bis(3-amino-1-ethylpropyl)-2-methyl-1,5-pentandiamin;
- Polyamine mit ein oder zwei sekundären Aminogruppen, insbesondere Produkte aus der reduktiven Alkylierung von primären aliphatischen Polyaminen mit Aldehyden oder Ketonen, insbesondere N¹-Benzyl-1,2-propandiamin, N¹-(4-Methoxybenzyl)-1,2-propandiamin, N-Benzyl-1,3-bis(aminomethyl)benzol, N,N'-Dibenzyl-1,3-bis(aminomethyl)benzol, N-2-Ethylhexyl-1,3-bis(aminomethyl)benzol, N,N'-Bis(2-ethylhexyl)-1,3-bis(aminomethyl)benzol, oder partiell styrolisierte Polyamine wie insbesondere partiell styrolisiertes MXDA (erhältlich als Gaskamine® 240 von Mitsubishi Gas Chemical);
- aromatische Polyamine, wie insbesondere m- und p-Phenylendiamin, 4,4'-, 2,4' und/oder 2,2'-Diaminodiphenylmethan, 3,3'-Dichloro-4,4'-diaminodiphenylmethan (MOCA), 2,4- und/oder 2,6-Toluylendiamin, Mischungen von 3,5-Dimethylthio-2,4- und -2,6-toluylendiamin (erhältlich als Ethacure® 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink® von Air Products), 1,2-Bis(2-aminophenylthio)ethan, 2-Methylpropyl-(4-chloro-3,5-diaminobenzoat) oder tert.Butyl-(4-chloro-3,5-diaminobenzoat);
- Addukte der genannten Polyamine mit Epoxiden oder Epoxidharzen, insbesondere Addukte mit Diepoxiden im Molverhältnis von ungefähr 2/1, Addukte mit Monoepoxiden im Molverhältnis von ungefähr 1/1, oder Umsetzungsprodukte aus Aminen und Epichlorhydrin, insbesondere jenes von 1,3-Bis(aminomethyl)benzol, kommerziell erhältlich als Gaskamine® 328 (von Mitsubishi Gas Chemical;
- Polyamidoamine, insbesondere Umsetzungsprodukte aus einer ein- oder mehrwertigen Carbonsäure, beziehungsweise deren Ester oder Anhydride, insbesondere einer Dimerfettsäure, mit einem im stöchiometrischen Überschuss eingesetzten aliphatischen, cycloaliphatischen oder aromatischen Polyamin, insbesondere einem Polyalkylenamin wie beispielsweise DETA oder TETA, insbesondere die kommerziell erhältlichen Polyamidoamine Versamid® 100, 125, 140 oder 150 (von Cognis), Aradur® 223, 250 oder 848 (von Huntsman), Euretek® 3607 oder 530 (von Huntsman) oder Beckopox® EH 651, EH 654, EH 655, EH 661 oder EH 663 (von Cytec); oder
- Phenalkamine, d.h. Mannich-Basen von Cardanol (langkettigen Alk(en)ylphenolen und -resorcinen gewonnen durch thermische Behandlung von Cashewschalenöl-Extrakten, enthaltend als Hauptkomponente 3-(Pentadeca-8,11,14-trienyl)phenol), insbesondere die kommerziell erhältlichen Phenalkamine Cardolite® NC-541, NC-557, NC-558, NC-566, Lite 2001, Lite 2002, NX-5607 oder NX-5608 (von Cardolite), Aradur® 3440, 3441, 3442 oder 3460 (von Huntsman) oder Beckopox® EH 614, EH 621, EH 624, EH 628 oder EH 629 (von Cytec).

Bevorzugt ist das weitere Amin ausgewählt aus der Gruppe bestehend aus 2-Butyl-2-ethyl-1,5-pentandiamin (C11-Neodiamin), 2,2(4),4-Trimethylhexamethylendiamin (TMD), 1,12-Dodecandiamin, Bis(4-aminocyclohexyl)methan (H₁₂-MDA), 1-Amino-3-aminomethyl-3,5,5-trimethylcyclohexan (Isophorondiamin oder IPDA), 1,3-Bis(aminomethyl)cyclohexan, 1,4-Bis(aminomethyl)cyclohexan, 2,5(2,6)-Bis(aminomethyl)bicyclo[2.2.1]heptan (NBDA), 3(4),8(9)-Bis-(aminomethyl)tricyclo[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro-[5.5]undecan, 1,3-Bis(aminomethyl)benzol (MXDA), Ethergruppen-haltigen aliphatischen primären Di- und Triaminen, N¹-Benzyl-1,2-propandiamin, N¹-(4-Methoxybenzyl)-1,2-propandiamin, N-Benzyl-1,3-bis(aminomethyl)benzol, N,N'-Dibenzyl-1,3-bis(aminomethyl)benzol, N-2-Ethylhexyl-1,3-bis(aminomethyl)benzol, N,N'-Bis(2-ethylhexyl)-1,3-bis(aminomethyl)benzol, partiell styrolisiertem 1,3-Bis(aminomethyl)benzol und Addukten aus (i) mindestens einem Polyamin mit mindestens drei gegenüber Epoxidgruppen reaktiven Aminwasserstoffen mit (ii) mindestens einem Epoxid.

Bevorzugt sind TMD, IPDA, NBDA oder MXDA. Diese weiteren Amine sind wenig flüchtig und ermöglichen zusammen mit dem Amin der Formel (I) gut verarbeitbare, schnell aushärtende Epoxidharz-Produkte von hoher Härte und Beständigkeit mit schönen Oberflächen.

Bevorzugt sind weiterhin Ethergruppen-haltige aliphatische primäre Di- und Triamine, insbesondere Polyoxyalkylendi- oder -triamine mit einem mittleren Molekulargewicht im Bereich von 200 bis 500 g/mol, insbesondere Jeffamine® D-230 oder Jeffamine® T-403 (beide von Huntsman), oder cycloaliphatische Ethergruppen-haltige Diamine aus der Propoxylierung und nachfolgenden Aminierung von 1,4-Dimethylolcyclohexan, insbesondere Jeffamine® RFD-270 (von Huntsman). Diese weiteren Amine ermöglichen zusammen mit dem Amin der Formel (I) gut verarbeitbare Epoxidharz-Produkte mit mit geringer Sprödigkeit und einer zuverlässigen Aushärtung zu hoher Endhärte ohne sogenanntes "Einfrieren". Mit "Einfrieren" wird das Phänomen bezeichnet, dass eine Epoxidharz-Zusammensetzung bei einer gegebenen Temperatur nach anfänglich guter Härteentwicklung nicht zu der erwarteten Endhärte aushärtet, sondern die Aushärtung bei einer geringeren Härte stehenbleibt. Solche Effekte treten insbesondere bei tiefen Aushärtungstemperaturen auf.

Bevorzugt sind weiterhin N¹-Benzyl-1,2-propandiamin, N¹-(4-Methoxybenzyl)-1,2-propandiamin, N-Benzyl-1,3-bis(aminomethyl)benzol, N,N'-Dibenzyl-1,3-bis(aminomethyl)benzol, N-2-Ethylhexyl-1,3-bis(aminomethyl)benzol, N,N'-Bis-(2-ethylhexyl)-1,3-bis(aminomethyl)benzol oder partiell styrolisiertes 1,3-Bis-(aminomethyl)benzol, insbesondere das kommerziell erhältliche Gaskamine® 240 (von Mitsubishi Gas Chemical). Diese weiteren Amine ermöglichen zusammen mit dem Amin der Formel (I) besonders niedrigviskose und somit gut verarbeitbare Epoxidharz-Produkte mit besonders schönen Oberflächen.

Bevorzugt sind weiterhin Addukte aus (i) mindestens einem Polyamin mit mindestens drei gegenüber Epoxidgruppen reaktiven Aminwasserstoffen mit (ii) mindestens einem Epoxid.

Als Polyamin für ein solches Addukt bevorzugt sind die vorgängig genannten Polyamine mit mindestens drei gegenüber Epoxidgruppen reaktiven Aminwasserstoffen, oder kleinere Polyamine wie insbesondere Ethylendiamin, die isomeren Propylendiamine oder die isomeren Butylendiamine.

Als Epoxid für ein solches Addukt bevorzugt sind Diepoxide, wie insbesondere Bisphenol-A- oder -F- oder -A/F-diglycidylether, Poly-1,2-propylenoxiddiglycidylether oder Monoepoxide. Besonders bevorzugt sind aromatische Monoepoxide, insbesondere Kresylglycidylether, tert.Butylphenylglycidylether oder der Glycidylether von Cardanol. Besonders bevorzugt ist Kresylglycidylether. Als Kresylglycidylether geeignet sind alle isomeren Kresylglycidylether oder Gemische davon, insbesondere kommerziell erhältiche Typen wie insbesondere Araldite® DY-K (von Huntsman), Polypox™ R6 (von Dow), Heloxy™ KR (von Hexion) oder Erisys® GE-10 (von CVC Spec. Chem.).

Das Addukt wird bevorzugt hergestellt durch langsames Zudosieren des Epoxids zu vorgelegtem Polyamin, wobei die Temperatur der Reaktanden bevorzugt im Bereich von 40 bis 120 °C, insbesondere 50 bis 110 °C, gehalten wird.

Bevorzugt sind Addukte aus (i) mindestens einem Polyamin mit mindestens drei gegenüber Epoxidgruppen reaktive Aminwasserstoffen mit (ii) mindestens einem aromatischen Monoepoxid, welche im Molverhältnis von ungefähr 1/1 umgesetzt sind. Während der Umsetzung kann das Polyamin im Überschuss vorhanden gewesen und nach der Umsetzung mittels Destillation entfernt worden sein.

Für ein solches Addukt ist das Polyamin bevorzugt ausgewählt aus der Gruppe bestehend aus Ethylendiamin, 1,2-Propylendiamin, 1,3-Propylendiamin, 1,4-Butylendiamin, 1,3-Butylendiamin, 1,2-Butylendiamin, 2,3- Butylendiamin, 2-Methyl-1,3-propandiamin, DAMP, 2,2-Dimethyl-1,3-propandiamin, 1,5-Pentandiamin, MPMD, 1,6-Hexandiamin, 2,5-Dimethyl-1,6-hexandiamin, TMD, 1,2-Diaminocyclohexan, 1,3-Diaminocyclohexan, 1,4-Diaminocyclohexan, IPDA, 2-Methyl-1,3-diaminocyclohexan und 4-Methyl-1,3-diaminocyclohexan und Mischungen davon, 1,3-Bis(aminomethyl)cyclohexan, 1,4-Bis(aminomethyl)cyclohexan, 1,3-Bis(aminomethyl)benzol, Bis(2-aminoethyl)ether, 3,6-Dioxaoctan-1,8-diamin, DETA, TETA, DPTA, N3-Amin, N4-Amin und BHMT.

Für ein solches Addukt ist das aromatische Monoepoxid bevorzugt ein Kresylglycidylether.

Besonders bevorzugt ist ein Addukt aus 1,2-Propylendiamin mit Kresylglycidylether, welches hergestellt ist mit einem Überschuss 1,2-Propylendiamin und nachfolgender Entfernung des Überschusses mittels Destillation.

Weiterhin besonders bevorzugt ist ein Addukt aus 1,5-Diamino-2-methylpentan mit Kresylglycidylether, welches entweder hergestellt ist mit einem Überschuss 1,5-Diamino-2-methylpentan und nachfolgender Entfernung des Überschusses mittels Destillation, oder mit einem leichten Überschuss an Kresylglycidylether. Weiterhin besonders bevorzugt ist ein Addukt aus 2,2(4),4-Trimethylhexamethylendiamin mit Kresylglycidylether, welches hergestellt ist mit einem leichten Überschuss an 2,2(4),4-Trimethylhexamethylendiamin.

Der Begriff "Überschuss" bezieht sich bei diesen besonders bevorzugten Addukten nicht auf die Reaktivgruppen, sondern auf das Molverhältnis zwischen dem Polyamin und dem Kresylglyciylether.

Diese besonders bevorzugten Addukte sind vergleichsweise niedrigviskos, weisen eine besonders gute Verträglichkeit und Reaktivität mit den üblichen Epoxidharz-Zusammensetzungen auf, neigen kaum zu Blushing-Effekten und ermöglichen ausgehärtete Filme von hohem Glanz und hoher Härte. Zusammen mit dem Amin der Formel (I) wird ein besonders rascher Aufbau der Festigkeit bei guter Verarbeitbarkeit erreicht.

Als weiteres Amin ganz besonders bevorzugt ist N¹-Benzyl-1,2-propandiamin. Ein Härter, welcher neben dem Amin der Formel (I) als weiteres Amin N¹-Benzyl-1,2-propandiamin enthält ist vor allem auch deshalb besonders interessant, weil ein Amin der Formel (I) aus reduktiver Alkylierung und N¹-Benzyl-1,2-propandiamin auf einfache Weise zusammen hergestellt werden können, indem 1,2-Propylendiamin mit einer Mischung aus Benzaldehyd und dem Aldehyd oder Keton der Formel (II), insbesondere Salicylaldehyd, reduktiv alkyliert wird. Dabei kann durch das gewählte Verhältnis zwischen N¹-Benzyl-1,2-propandiamin und dem Amin der Formel (I) eine gewünschte Kombination zwischen niedriger Viskosiät und hoher Reaktivität eingestellt werden.

Bevorzugt ist ein Molverhältnis zwischen N¹-Benzyl-1,2-propandiamin und dem Amin der Formel (I) im Bereich von 10/1 bis 1/10, insbesondere 5/1 bis 1/5.

Als Additiv geeignet sind insbesondere Beschleuniger, Lösemittel, Verdünner, Extender, Rheologie-Modifizierer oder oberflächenaktive Substanzen wie insbesondere Netzmittel, Verlaufsmittel, Entlüftungsmittel oder Entschäumer.

Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; tertiäre Amine wie insbesondere 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen. Als Beschleuniger bevorzugt sind Säuren, tertiäre Amine oder Mannich-Basen.

Am meisten bevorzugt ist Salicylsäure oder 2,4,6-Tris(dimethylaminomethyl)-phenol oder eine Kombination davon.

Bevorzugt enthält der Härter keinen oder nur einen geringen Gehalt an Beschleunigern. Durch das Amin der Formel (I) ist der Härter auch ohne zusätzliche Beschleuniger sehr reaktiv. Bevorzugt ist der Härter insbesondere weitgehend frei von 2,4,6-Tris(dimethylaminomethyl)phenol, welches im Härter einen unangenehmen Geruch und Emission verursacht und zu Vergilbung der ausgehärteten Zusammensetzung führen kann. Bevorzugt enthält der Härter weniger als 1 Gewichts-%, besonders bevorzugt weniger als 0.5 Gewichts-%, insbesondere weniger als 0.1 Gewichts-% an 2,4,6-Tris(dimethylaminomethyl)phenol.

Als Lösemittel, Verdünner oder Extender, im folgenden kollektiv Verdünner genannt, geeignet sind insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie insbesondere Solvesso®-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide. Bevorzugt sind Benzylalkohol, Dodecylphenol, tert.Butylphenol, styrolisiertes Phenol, ethoxyliertes Phenol oder phenolgruppenhaltige aromatische Kohlenwasserstoffharze, insbesondere die Novares®-Typen LS 500, LX 200, LA 300 oder LA 700 (von Rütgers).

Bevorzugt enthält der Härter keinen oder nur einen geringen Gehalt an Verdünnern. Bevorzugt enthält der Härter maximal 5 Gewichts-% Verdünner.

Geeignete Rheologie-Modifizierer sind insbesondere Verdicker oder Antiabsetzmittel.

Der Härter kann weitere gegenüber Epoxidgruppen reaktive Substanzen enthalten, beispielsweise Monoamine wie Hexylamin oder Benzylamin, oder Mercaptogruppen aufweisende Verbindungen, insbesondere die Folgenden:
- flüssige Mercaptan-terminierte Polysulfid-Polymere, bekannt unter dem Markennamen Thiokol® (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 oder LP-2; sowie weiterhin bekannt unter dem Markennamen Thioplast® (von Akzo Nobel), insbesondere die Typen G 10, G 112, G 131, G 1, G 12, G 21, G 22, G 44 oder G 4;
- Mercaptan-terminierte Polyoxyalkylen-Ether, erhältlich beispielsweise durch Umsetzung von Polyoxyalkylendi- oder -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure® (von Cognis), insbesondere die Typen WR-8, LOF oder 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri(3-mercaptopropionat) oder Glykoldi-(3-mercaptopropionat), oder Veresterungsprodukte von Polyoxyalkylendiolen oder -triolen, ethoxyliertem Trimethylolpropan oder Polyester-Diolen mit Thiocarbonsäuren wie Thioglykolsäure oder 2- oder 3-Mercaptopropionsäure; oder
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) oder Ethandithiol.

Bevorzugt enthält der Härter das Amin der Formel (I) in einer solchen Menge, dass 1 bis 95 %, besonders bevorzugt 5 bis 90 %, insbesondere 10 bis 80 %, der im Härter vorhandenen Aminwasserstoffe aus dem Amin der Formel (I) stammen.

Ein weiterer Gegenstand der Erfindung ist eine Epoxidharz-Zusammensetzung umfassend
- eine Harz-Komponente enthaltend mindestens ein Epoxidharz und
- eine Härter-Komponente enthaltend mindestens ein Amin der Formel (I) wie vorgängig beschrieben.

Bevorzugt ist die Härter-Komponente ein Härter enthaltend mindestens ein Amin der Formel (I) und mindestens ein weiteres Amin und/oder mindestens ein Additiv, wie vorgängig beschrieben.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.

Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf.

Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)methan, 2,2-Bis(4-hydroxy-3-methylyphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1 -Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylenbis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂-bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz in der Harz-Komponente bevorzugt ist ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman oder Momentive erhältlich sind. Diese Flüssigharze weisen eine für Epoxidharze niedrige Viskosität und im ausgehärteten Zustand gute Eigenschaften als Beschichtung auf. Sie können Anteile von Bisphenol A-Festharz oder Bisphenol-F-Novolaken enthalten.

Die Harz-Komponente kann einen Reaktivverdünner, insbesondere einen mindestens eine Epoxidgruppe aufweisenden Reaktivverdünner, enthalten. Als Reaktivverdünner geeignet sind insbesondere die Glycidylether von ein- oder mehrwertigen Phenolen oder aliphatischen oder cycloaliphatischen Alkoholen, wie insbesondere die bereits genannten Polyglycidylether von Di- oder Polyolen, oder weiterhin Phenylglycidylether, Kresylglycidylether, Benzylglycidylether, p-n-Butylphenylglycidylether, p-tert.Butylphenylglycidylether, Nonylphenylglycidylether, Allylglycidylether, Butylglycidylether, Hexylglycidylether, 2-Ethylhexylglycidylether, oder Glycidylether von natürlichen Alkoholen wie insbesondere C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, und/oder eine Reduktion der Glasübergangstemperatur und/oder der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner oder Extender, wie insbesondere die bereits genannten Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate oder Reaktivgruppen-aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere, Sulfonamid-modifizierte Melamine oder gereinigte Montan-Wachse;
- anorganische oder organische Füllstoffe, insbesondere gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und/oder Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht oder UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat oder Bisphenol-A-bis(diphenylphosphat), Tris(chloroethyl)phosphat, Tris(chloropropyl)phosphat oder Tris(dichloroisopropyl)phosphat, Tris[3-bromo-2,2-bis-(bromomethyl)propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine;
- oberflächenaktive Substanzen, insbesondere Netzmittel, Verlaufsmittel, Entlüftungsmittel oder Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und/oder Beschleuniger.

Bevorzugt enthält die Epoxidharz-Zusammensetzung keinen oder nur einen geringen Gehalt an Verdünnern, bevorzugt maximal 5 Gewichts-%, insbesondere maximal 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.

Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Die beiden Komponenten der Epoxidharz-Zusammensetzung werden jeweils in einem eigenen Gebinde gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1/10 bis 10/1.

Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.

Mit der Vermischung der beiden Komponenten beginnt die Aushärtung durch chemische Reaktion, wie vorgängig beschrieben. Die Aushärtung erfolgt insbesondere bei Umgebungstemperatur. Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.

Aus der Aushärtung der Zusammensetzung wird eine ausgehärtete Epoxidharz-Zusammensetzung erhalten.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips oder Natursteine wie Granit oder Marmor;
- Metalle oder Legierungen wie Aluminium, Eisen, Stahl oder Buntmetalle, oder oberflächenveredelte Metalle oder Legierungen wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe oder weitere sogenannte Polymer-Composites;
- Kunststoffe, insbesondere Hart- oder Weich-PVC, ABS, Polycarbonat (PC), Polyamid (PA), Polyester, PMMA, Epoxidharze, PUR, POM, PO, PE, PP, EPM oder EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) oder Sheet Moulding Compounds (SMC);
- beschichtete Substrate, wie pulverbeschichtete Metalle oder Legierungen;
- Farben oder Lacke.

Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundmatrix für Faserverbundwerkstoffe (Composites) wie insbesondere CFK oder GFK, oder als Vergussmasse, Dichtstoff, Klebstoff, Belag, Beschichtung, Anstrich, Lack, Versiegelung, Grundierung oder Primer, sowie als zementäres Produkt wie insbesondere Reparaturmörtel oder Vergussmörtel (Grout).

Insbesondere verwendbar ist sie als Vergussmasse, beispielsweise als Elektrovergussmasse, oder als Klebstoff, insbesondere als Karrosserieklebstoff, Sandwichelementklebstoff, Halbschalenklebstoff für Rotorblätter von Windkraftanlagen, Brückenelementklebstoff oder Verankerungsklebstoff.

Insbesondere verwendbar ist sie weiterhin als Belag, Beschichtung, Anstrich, Lack, Versiegelung, Grundierung oder Primer für Bau- und Industrieanwendungen, insbesondere als Bodenbelag oder Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden.

Insbesondere verwendbar ist sie weiterhin als Voranstrich, Haftanstrich, Korrosionsschutz-Primer oder zur Hydrophobierung von Oberflächen.

Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft wird die beschriebene Epoxidharz-Zusammensetzung als Beschichtung verwendet.

Ein weiterer Gegenstand der Erfindung ist somit eine Beschichtung, erhalten aus einer Epoxidharz-Zusammensetzung wie vorgängig beschrieben.

Als Beschichtung werden dabei flächig aufgebrachte Beläge aller Art verstanden, insbesondere Anstriche, Lacke, Versiegelungen, Grundierungen oder Primer, wie vorgängig beschrieben, oder Bodenbeläge oder Schutzbeschichtungen, insbesondere auch solche für schweren Korrosionsschutz.

Besonders vorteilhaft wird die beschriebene Epoxidharz-Zusammensetzung in emissionsarmen Beschichtungen mit Öko-Gütesiegeln, beispielsweise nach Emicode (EC1 Plus), AgBB, DIBt, Der Blaue Engel, AFSSET, RTS (M1) und US Green Building Council (LEED), verwendet.

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende oder thixotropierte Beschichtung auf überwiegend ebene Flächen oder als Anstrich appliziert wird. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 10'000 mPa·s, bevorzugt im Bereich von 300 bis 7'500 mPa·s, besonders bevorzugt im Bereich von 300 bis 4'000 mPa·s, insbesondere im Bereich von 300 bis 2'000 mPa·s, am meisten bevorzugt im Bereich von 300 bis 1'000 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann auch mit einem Pinsel oder Roller oder als Spritzapplikation erfolgen, beispielsweise als Korrosionsschutzbeschichtung auf Stahl.

Bei der Aushärtung entstehen typischerweise weitgehend klare, glänzende und nichtklebrige Filme von hoher Härte, welche eine gute Haftung zu verschiedensten Substraten aufweisen.

Die Filme sind besonders beständig gegenüber Feuchtigkeit und diversen Chemikalien, was durch die in die Matrix eingebauten Phenolgruppen begünstigt wird.

Aus der Anwendung der Epoxidharz-Zusammensetzung entsteht ein Artikel umfassend die ausgehärtete Zusammensetzung wie beschriebenen.

Die beschriebene Epoxidharz-Zusammensetzung zeichnet sich durch vorteilhafte Eigenschaften aus. Sie ist Phenol-frei und auch ohne Verdünner gut verarbeitbar. Sie härtet auch ohne Beschleuniger sowohl bei Raumtemperatur als auch bei tieferen Umgebungstemperaturen sehr rasch aus und bildet dabei eine hohe Härte und eine schöne Oberfläche aus und vergilbt kaum.

Ein weiterer Gegenstand der Erfindung ist eine Methode zum Beschleunigen der Aushärtung einer Epoxidharz-Zusammensetzung, indem ein Amin der Formel (I) wie vorgängig beschrieben zugegeben wird.

Die Epoxidharz-Zusammensetzung umfasst dabei insbesondere eine Harz-Komponente und eine Härter-Komponente bzw. einen Härter. Bevorzugt wird das Amin der Formel (I) der Härter-Komponente bzw. dem Härter zugegeben. Die Härter enthält bevorzugt mindestens ein Amin wie die vorgängig genannten weiteren Amine, insbesondere mindestens eines der vorgängig genannten bevorzugten Amine.

Bevorzugt wird das Amin der Formel (I) in einer solchen Menge zugegeben, dass 1 bis 80 %, insbesondere 2 bis 50 %, insbesondere 3 bis 30 %, der im Härter vorhandenen Aminwasserstoffe aus dem Amin der Formel (I) stammen.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" wird eine Temperatur von 23±1 °C und eine relative Luftfeuchtigkeit von 50±5% bezeichnet. "NK" steht für "Normklima".

### Beschreibung der Messmethoden:

**Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).

Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.

Die **Aminzahl** wurde durch Titration bestimmt (mit 0.1N HClO₄ in Essigsäure gegen Kristallviolett).

### Herstellung von Aminen der Formel (I)

### Amin A1: Reaktionsprodukt enthaltend N¹-(2-Hydroxybenzyl)-1,2-propandiamin

In einem Rundkolben wurden 92.67 g (1.25 mol) 1,2-Propylendiamin unter Stickstoffatmosphäre bei Raumtemperatur vorgelegt. Unter gutem Rühren wurde langsam eine Lösung von 30.53 g (0.25 mol) Salicylaldehyd in 800 ml Isopropanol dazugetropft und 2 Stunden nachgerührt. Danach wurde die Reaktionsmischung bei einem Wasserstoff-Druck von 90 bar, einer Temperatur von 95 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 5.2 Pa·s bei 20 °C und einer Aminzahl von 573 mg KOH/g.
FT-IR: 3286, 3044, 2958, 2921, 2850, 2725, 2588, 1589, 1455, 1412, 1378, 1253, 1184, 1150, 1102, 1035, 931, 842, 772.

### Amin A2: Reaktionsprodukt enthaltend N¹-(2-Hydroxybenzyl)-1,2-propandiamin und N¹-Benzyl-1,2-propandiamin

In einem Rundkolben wurden 92.67 g (1.25 mol) 1,2-Propylendiamin unter Stickstoffatmosphäre bei Raumtemperatur vorgelegt. Unter gutem Rühren wurde langsam eine Lösung von 6.10 g (0.05 mol) Salicylaldehyd und 21.22 g (0.20 mol) Benzaldehyd in 500 ml Isopropanol dazugetropft und 2 Stunden nachgerührt. Danach wurde die Reaktionsmischung bei einem Wasserstoff-Druck von 85 bar, einer Temperatur von 95 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbe Flüssigkeit mit einer Viskosität von 0.1 Pa·s bei 20 °C und einer Aminzahl von 630 mg KOH/g.
FT-IR: 3025, 2957, 2820, 1590, 1493, 1452, 1373, 1256, 1105, 1027, 828, 733, 699.

### Amin A3: Reaktionsprodukt enthaltend 2,4,6-Tris-(((2-aminopropyl)amino)methyl)phenol

In einem Rundkolben mit Rückflusskühler (Kühlwassertemperatur 12 °C) wurden 148.26 g (2.00 mol) 1,2-Propylendiamin und 43.8 g (0.17 mol) 2,4,6-Tris-(dimethylaminomethyl)phenol (Ancamine® K 54, von Air Products) unter Stickstoffatmosphäre vorgelegt und auf 130 °C aufgewärmt. Während 5 Stunden wurde die Temperatur gehalten und 1,2-Propylendiamin rückflussiert, während freigesetztes Dimethylamin in einer an den Rückflusskühler anschliessenden Kühlfalle aufgefangen wurde. Anschliessen wurde die Reaktionsmischung im Vakuum bei 65 °C eingeengt. Erhalten wurde eine gelbliche Flüssigkeit mit einer Viskosität von 82.3 Pa·s bei 40 °C und einer Aminzahl von 933 mg KOH/g.
FT-IR: 3285, 2954, 2813, 1608, 1450, 1405, 1356, 1294, 1250, 1175, 1146, 1103, 1026, 988, 875

### Amin A4: Reaktionsprodukt enthaltend 2,4,6-Tris-(((2-aminopropyl)amino)methyl)phenol

In einem Rundkolben mit Rückflusskühler (Kühlwassertemperatur 12 °C) wurden 25.94 g (0.35 mol) 1,2-Propylendiamin und 29.49 g (0.11 mol) 2,4,6-Tris-(dimethylaminomethyl)phenol (Ancamine® K 54, von Air Products) unter Stickstoffatmosphäre vorgelegt und auf 135 °C aufgewärmt. Während 5 Stunden wurde die Temperatur gehalten und 1,2-Propylendiamin rückflussiert, während freigesetztes Dimethylamin in einer Kühlfalle, welche nach dem Rückflusskühler angebracht war, aufgefangen wurde. Anschliessen wurde die Reaktionsmischung im Vakuum bei 65 °C eingeengt. Erhalten wurde eine gelbliche Flüssigkeit mit einer Viskosität von 3.9 Pa·s bei 40 °C und einer Aminzahl von 865 mg KOH/g.
FT-IR: 3275, 2955, 2816, 1607, 1455, 1371, 1294, 1250, 1147, 1105, 837, 787.

### Herstellung von Aminen für Vergleichszwecke

### Amin C1: Reaktionsprodukt enthaltend N¹-Benzyl-1,2-propandiamin

In einem Rundkolben wurden 22.1 g (0.3 mol) 1,2-Propylendiamin unter Stickstoffatmosphäre bei Raumtemperatur vorgelegt. Unter gutem Rühren wurde langsam eine Lösung von 31.8 g (0.3 mol) Benzaldehyd in 500 ml Isopropanol dazugetropft und 30 min. nachgerührt. Danach wurde die Reaktionsmischung bei einem Wasserstoff-Druck von 85 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung wurde im Vakuum bei 65 °C eingeengt. Erhalten wurde eine gelbliche Flüssigkeit mit einer Viskosität von 19 mPa·s bei 20 °C und einer Aminzahl von 574 mg KOH/g.
FT-IR: 3026, 2956, 2818, 1601, 1494, 1452, 1373, 1115, 1073, 1028, 826, 732, 696.

### Amin C2: Reaktionsprodukt enthaltend N¹-(2-Hydroxybenzyl)-4-methyl-1,5-pentandiamin

In einem Rundkolben wurden 23.24 g (0.20 mol) 1,5-Diamino-2-methylpentan (Dytek® A, von Invista) unter Stickstoffatmosphäre bei Raumtemperatur vorgelegt. Unter gutem Rühren wurde langsam eine Lösung von 24.42 g (0.20 mol) Salicylaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbe Flüssigkeit mit einer Viskosität von 2.3 Pa·s bei 20 °C und einer Aminzahl von 506 mg KOH/g.
FT-IR: 2924, 2850, 1589, , 1455, 1411, 1255, 1101, 930, 842, 748, 719.

### Amin C3: Reaktionsprodukt enthaltend N¹-(2-Hydroxybenzyl)-3,3(5),5-trimethyl-1,6-hexandiamin

In einem Rundkolben wurden 15.82 g (0.10 mol) 2,2(4),4-Trimethylhexamethylendiamin (Vestamin® TMD, von Evonik) unter Stickstoffatmosphäre bei Raumtemperatur vorgelegt. Unter gutem Rühren wurde langsam eine Lösung von 12.21 g (0.10 mol) Salicylaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine dunkelgelbe Flüssigkeit mit einer Viskosität von 4.4 Pa·s bei 20 °C und einer Aminzahl von 414 mg KOH/g.
FT-IR: 3045, 2952, 2912, 2868, 1589, 1470, 1256, 1101, 931, 844, 748, 720.

### Amin C4: Reaktionsprodukt enthaltend N-(2-Hydroxybenzyl)-1,3-bis(aminomethyl)benzol

In einem Rundkolben wurden 27.24 g (0.20 mol) 1,3-Bis(aminomethyl)benzol (MXDA, von Mitsubishi Gas Chemicals) unter Stickstoffatmosphäre bei Raumtemperatur vorgelegt. Unter gutem Rühren wurde langsam eine Lösung von 24.42 g (0.20 mol) Salicylaldehyd in 250 ml Isopropanol dazugetropft und 2 Stunden nachgerührt. Danach wurde die Reaktionsmischung bei einem Wasserstoff-Druck von 80 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 22.3 Pa·s bei 20 °C und einer Aminzahl von 463 mg KOH/g.
FT-IR: 3022, 2846, 2721, 2613, 1587, 1454, 1254, 1082, 843, 747, 699.

### Amin C5: Reaktionsprodukt enthaltend N¹-(2-Hydroxybenzyl)-4,7-diaza-1,10-decandiamin

In einem Rundkolben wurden 34.85 g (0.20 mol) N,N'-Bis(3-aminopropyl)ethylendiamin (N4-Amine, von BASF) unter Stickstoffatmosphäre bei Raumtemperatur vorgelegt. Unter gutem Rühren wurde langsam eine Lösung von 24.42 g (0.20 mol) Salicylaldehyd in 400 ml Isopropanol dazugetropft und 2 Stunden nachgerührt. Danach wurde die Reaktionsmischung bei einem Wasserstoff-Druck von 85 bar, einer Temperatur von 90 °C und einem Fluss von 4 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung im Vakuum bei 65 °C eingeengt. Erhalten wurde eine gelbliche Flüssigkeit mit einer Viskosität von 2.5 Pa·s bei 20 °C und einer Aminzahl von 794 mg KOH/g.
FT-IR: 3287, 2927, 2837, 1630, 1581, 1496, 1458, 1277, 1150, 1115, 953, 836, 746, 736.

### Herstellung von Härtern und Epoxidharz-Zusammensetzungen

Für jedes Beispiel wurden die in den Tabellen 1 bis 2 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.

Ebenso wurden die in den Tabellen 1 bis 2 angegebenen Inhaltsstoffe der Harz-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C bestimmt **("Viskosität (10')").**

Ein erster Film wurde in einer Schichtdicke von 500 µm auf eine Glasplatte aufgezogen und dieser Normklima gelagert bzw. ausgehärtet. An diesem Film wurde die **Königshärte** (Pendelhärte nach König, gemessen nach DIN EN ISO 1522) nach 1 Tag ("Königshärte (NK) (1d)"), nach 2 Tagen ("Königshärte (NK) (2d)"), nach 4 Tagen ("Königshärte (NK) (4d)"), nach 7 Tagen ("Königshärte (NK) (7d)") und nach 14 Tagen ("Königshärte (NK) (14d)") bestimmt. Nach 14 Tagen wurde der Aspekt des Films beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.

Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 3 Wochen im NK gelagert, beziehungsweise ausgehärtet. Anschliessend wurde der Aspekt dieses Films beurteilt (in den Tabellen mit "Aspekt (8°/80%)" bezeichnet), auf die gleiche Weise wie für den Aspekt (NK) beschrieben. Am so ausgehärteten Filmen wurde wiederum die Königshärte bestimmt, jeweils nach 7 Tagen bei 8 °C und 80 % relativer Feuchtigkeit ("Königsh. (7d 8°/80%)"), dann nach weiteren 2 Tagen im NK ("Königsh. (7d 8°/80%) (+2d NK)") bzw. 7 Tagen im NK ("Königsh. (7d 8°/80%) (+7d NK)").

Als Mass für die Vergilbung wurde weiterhin die Farbveränderung nach Belastung in einem Bewitterungstester bestimmt. Dazu wurde ein weiterer Film in einer Schichtdicke von 500 µm auf eine Glasplatte aufgezogen und dieser im Normklima während 2 Wochen gelagert bzw. ausgehärtet und anschliessend in einem Bewitterungstester des Typs Q-Sun Xenon Xe-1 mit optischem Filter Q-SUN Daylight-Q und einer Xenon Lampe mit einer Lichtstärke von 0.51 W/m² bei 340 nm bei einer Temperatur von 65°C während 72 Stunden belastet **(Q-Sun (72h)).** Anschliessend wurde der Farbunterschied ΔE des so belasteten Films im Vergleich zum entsprechenden nicht belasteten Film mittels einem Colorimeter NH310 von Shenzen 3NH Technology Co. LTD, ausgerüstet mit Silicon Photoelectric Diode Detector, Light Source A, Color Space Measurement Interface CIE L*a*b*C*H*, bestimmt. Ein hoher ΔE-Wert steht dabei für eine grossen Farbunterschied bzw. eine starke Vergilbung.

Die Resultate sind in den Tabellen 1 bis 2 angegeben.

Bei den Epoxidharz-Zusammensetzungen ***EZ-1*** bis ***EZ-4*** handelt es sich um erfindungsgemässe Beispiele. Bei den Epoxidharz-Zusammensetzungen ***Ref-1*** bis ***Ref-5*** handelt es sich um Vergleichsbeispiele.

**Verwendete Substanzen:**

| | |
|---|---|
| Araldite® GY 250: | Bisphenol-A-Diglycidylether, EEW ca. 187.5 g/Eq (von Huntsman) |
| Araldite® DY-E: | Monoglycidylether von C₁₂-bis C₁₄-Alkoholen, EEW ca 290 g/Eq (von Huntsman) |

**Tabelle 1: Zusammensetzung und Eigenschaften von EZ-1 bis EZ-4 und Ref-1.**

| **Zusammensetzungen** | | ***EZ-1*** | ***EZ-2*** | ***EZ-3*** | ***EZ-4*** | ***Ref-1*** |
|---|---|---|---|---|---|---|
| **Harz-Komp.:** | | | | | | |
| Araldite® GY-250 | | 167.2 | 167.2 | 167.2 | 167.2 | 167.2 |
| Araldite® DY-E | | 31.8 | 31.8 | 31.8 | 31.8 | 31.8 |

| **Härter-Komp.:** | | | | | | |
|---|---|---|---|---|---|---|
| Amin A1 | | 60.1 | - | - | - | - |
| Amin A2 | | - | - | - | 55.1 | - |
| Amin A3 | | - | 39.2 | - | - | - |
| Amin A4 | | - | - | 39.2 | - | - |
| Amin C1 | | - | - | - | - | 54.7 |
| Viskosität (10') [Pa·s] | | 1.75 | 7.43 | 2.93 | 0.56 | 0.46 |
| Königshärte [s] | (1d NK) | 84 | 85 | 94 | 52 | 5 |
| | (2d NK) | 147 | 104 | 108 | 126 | 25 |
| | (4d NK) | 171 | 129 | 120 | 165 | 40 |
| | (7d NK) | 195 | 148 | 126 | 193 | 73 |
| | (14d NK) | 207 | 171 | 150 | 210 | 75 |
| Aspekt (NK) | | schön, farblos | schön, farblos | leicht trüb, farblos | schön, farblos | schön, farblos |
| Königsh. [s] | (7d 8°/80%) | 65 | 56 | 27 | 46 | 12 |
| | (+2d NK) | 168 | 85 | 36 | 157 | 25 |
| | (+7d NK) | 203 | 111 | 39 | 197 | 39 |
| Aspekt (8°/80%) | | schön, farblos | matt, leichte Struktur, farblos | matt, leicht trüb, farblos | schön, farblos | schön, farblos |

**Tabelle 2: Zusammensetzung und Eigenschaften von EZ-1 und Ref-2 bis Ref-5.**

| **Beispiel** | | ***EZ-1*** | ***Ref-2*** | ***Ref-3*** | ***Ref-4*** | ***Ref-5*** |
|---|---|---|---|---|---|---|
| **Harz-Komp.:** | | | | | | |
| Araldite® GY-250 | | 167.2 | 167.2 | 167.2 | 167.2 | 167.2 |
| Araldite® DY-E | | 31.8 | 31.8 | 31.8 | 31.8 | 31.8 |

| **Härter-Komp.:** | | | | | | |
|---|---|---|---|---|---|---|
| Amin A1 | | 60.1 | - | - | - | - |
| Amin C2 | | - | 74.1 | - | - | - |
| Amin C3 | | - | - | 88.1 | - | - |
| Amin C4 | | - | - | - | 80.8 | - |
| Amin C5 | | - | - | - | - | 56.1 |
| Viskosität (10') [Pa·s] | | 1.75 | 1.68 | 2.37 | 3.78 | 2.63 |
| Königshärte [s] | (1d NK) | 84 | 60 | 31 | 150 | 74 |
| | (2d NK) | 147 | 108 | 71 | 182 | 87 |
| | (4d NK) | 171 | 138 | 110 | 184 | 87 |
| | (7d NK) | 195 | 155 | 153 | 193 | 95 |
| | (14d NK) | 207 | 189 | 175 | 208 | 102 |
| Aspekt (NK) | | schön, farblos | leichte Struktur, gelblich | schön, gelblich | leichte Struktur, gelblich | klebrig, matt, gelblich |
| Q-Sun (72h) ΔE | | 3.8 | 12.3 | 5.7 | 5.8 | 6.1 |
| Königsh. [s] | (7d 8°/80%) | 65 | 27 | 33 | 119 | 52 |
| | (+2d NK) | 168 | 45 | 95 | 171 | 95 |
| | (+7d NK) | 203 | 103 | 153 | 185 | 113 |
| Aspekt (8°/80%) | | schön, farblos | matt, leichte Struktur, gelblich | leicht matt, gelblich | matt, leichte Struktur, gelblich | trüb, klebrig, Struktur, gelblich |

## Patentansprüche

1. Amin der Formel (I), 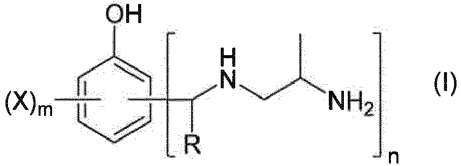 wobei
m für 0 oder 1 oder 2 steht,
n für 1 oder 2 oder 3 steht,
R für einen Wasserstoff-Rest oder Alkyl-Rest mit 1 bis 8 C-Atomen oder Phenyl-Rest steht, und
X für gleiche oder verschiedene Reste ausgewählt aus der Gruppe besteend aus Hydroxyl und Alkyl, Alkenyl und Alkoxy mit jeweils 1 bis 18 C-Atomen, welche gegebenenfalls Ether-Sauerstoff, Hydroxyl-Sauerstoff oder Amin-Stickstoff enthalten, oder für einen N-Pyrrolidinylmethyl- oder N-Piperidinylmethyl- oder N-Morpholinylmethyl-Rest steht.

2. Amin gemäss Anspruch 1, **dadurch gekennzeichnet, dass** m für 0 oder 1, n für 1 und R für einen Wasserstoff-Rest oder Methyl-Rest stehen.

3. Amin gemäss Anspruch 1, **dadurch gekennzeichnet, dass** (m+n) für 3, R für einen Wasserstoff-Rest und X für einen N,N-Dialkyl-Rest, welcher gegebenenfalls eine oder zwei Hydroxylgruppen enthält, oder für einen N-Alkyl-Rest, welcher gegebenenfalls eine Hydroxylgruppe oder ein oder zwei Amin-Stickstoffe enthält, oder für einen N-Pyrrolidinylmethyl- oder N-Piperidinylmethyl- oder N-Morpholinylmethyl-Rest stehen.

4. Verfahren zur Herstellung des Amins gemäss einem der Ansprüche 1 bis 3, **dadurch gekennzeichnet, dass** 1,2-Propylendiamin
- entweder unter reduktiver Alkylierung mit mindestens einem Aldehyd oder Keton der Formel (II) und Wasserstoff 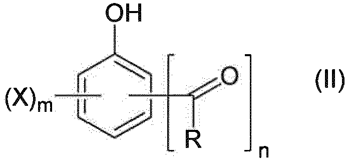
- oder unter Umaminierung mit einer Mannich-Base der Formel (III) 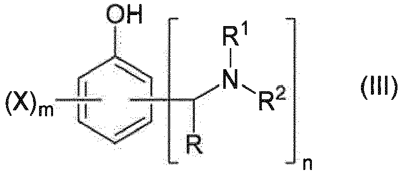 umgesetzt wird, wobei
R¹ und R² jeweils für gleiche oder verschiedene Alkyl-, Cycloalkyl-, oder Aralkyl-Reste mit 1 bis 4 C-Atomen, welche gegebenenfalls Ether-Sauerstoff oder Amin-Stickstoff enthalten, oder zusammen für einen Alkylen-Rest mit 4 bis 8 C-Atomen, welcher gegebenenfalls Ether-Sauerstoff oder Amin-Stickstoff enthält, stehen.

5. Verfahren gemäss Anspruch 4, **dadurch gekennzeichnet, dass** es mit einem Verhältnis zwischen der Anzahl 1,2-Propylendiamin-Moleküle und der Anzahl Aldehyd- oder Keto-Gruppen des Aldehyds oder Ketons der Formel (II) von mindestens 1.5/1 durchgeführt wird.

6. Verfahren gemäss einem der Ansprüche 4 oder 5, **dadurch gekennzeichnet, dass** es mit Salicylaldehyd oder 2'-Hydroxyacetophenon als Aldehyd oder Keton der Formel (II) durchgeführt wird.

7. Verfahren gemäss Anspruch 4, **dadurch gekennzeichnet, dass** es mit einem Verhältnis zwischen der Anzahl 1,2-Propylendiamin-Moleküle und der Anzahl Aminoalkyl-Substituenten der Mannich-Base der Formel (III) von mindestens 1/1 durchgeführt wird.

8. Verfahren gemäss einem der Ansprüche 4 oder 7, **dadurch gekennzeichnet, dass** es mit 2,4,6-Tris(N,N-dimethylaminomethyl)phenol als Mannich-Base der Formel (III) durchgeführt wird.

9. Verwendung von mindestens einem Amin gemäss einem der Ansprüche 1 bis 3 als Härter für Epoxidharze.

10. Härter für Epoxidharze enthaltend mindestens ein Amin gemäss einem der Ansprüche 1 bis 3 und mindestens ein weiteres Amin und/oder mindestens ein Additiv.

11. Härter gemäss Anspruch 10, **dadurch gekennzeichnet, dass** das weitere Amin ausgewählt ist aus der Gruppe bestehend aus 2-Butyl-2-ethyl-1,5-pentandiamin, 2,2(4),4-Trimethylhexamethylendiamin, 1,12-Dodecandiamin, Bis(4-aminocyclohexyl)methan, 1-Amino-3-aminomethyl-3,5,5-trimethylcyclohexan, 1,3-Bis(aminomethyl)cyclohexan, 1,4-Bis(aminomethyl)cyclohexan, 2,5(2,6)-Bis(aminomethyl)bicyclo[2.2.1]heptan, 3(4),8(9)-Bis(aminomethyl)tricyclo[5.2.1.0^{2,6}]decan, 1,4-Diamino-2,2,6-trimethylcyclohexan, 1,8-Menthandiamin, 3,9-Bis(3-aminopropyl)-2,4,8,10-tetraoxaspiro[5.5]undecan, 1,3-Bis(aminomethyl)benzol, Ethergruppen-haltigen aliphatischen primären Di- und Triaminen, N¹-Benzyl-1,2-propandiamin, N¹-(4-Methoxybenzyl)-1,2-propandiamin, N-Benzyl-1,3-bis(aminomethyl)-benzol, N,N'-Dibenzyl-1,3-bis(aminomethyl)benzol, N-2-Ethylhexyl-1,3-bis(aminomethyl)benzol, N,N'-Bis(2-ethylhexyl)-1,3-bis(aminomethyl)benzol, partiell styrolisiertem 1,3-Bis(aminomethyl)benzol und Addukten aus (i) mindestens einem Polyamin mit mindestens drei gegenüber Epoxidgruppen reaktiven Aminwasserstoffen mit (ii) mindestens einem Epoxid.

12. Härter gemäss Anspruch 11, **dadurch gekennzeichnet, dass** das weitere Amin N¹-Benzyl-1,2-propandiamin ist.

13. Epoxidharz-Zusammensetzung umfassend
- eine Harz-Komponente enthaltend mindestens ein Epoxidharz und
- eine Härter-Komponente enthaltend mindestens ein Amin gemäss einem der Ansprüche 1 bis 3.

14. Beschichtung erhalten aus einer Epoxidharz-Zusammensetzung gemäss Anspruch 13.

15. Methode zum Beschleunigen der Aushärtung einer Epoxidharz-Zusammensetzung, **dadurch gekennzeichnet, dass** ein Amin gemäss einem der Ansprüche 1 bis 3 zugegeben wird.

## Claims

1. Amine of the formula (I), 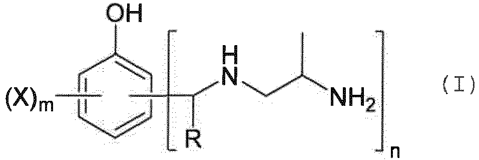 where
m is 0 or 1 or 2,
n is 1 or 2 or 3,
R is a hydrogen radical or alkyl radical having 1 to 8 carbon atoms or phenyl radical, and
X represents identical or different radicals selected from the group consisting of hydroxyl and alkyl, alkenyl and alkoxy having in each case 1 to 18 carbon atoms, and optionally containing ether oxygen, hydroxyl oxygen or amine nitrogen, or is an N-pyrrolidinylmethyl or N-piperidinylmethyl or N-morpholinylmethyl radical.

2. Amine according to Claim 1, **characterized in that** m is 0 or 1, n is 1, and R is a hydrogen radical or methyl radical.

3. Amine according to Claim 1, **characterized in that** (m+n) is 3, R is a hydrogen radical, and X is an N,N-dialkyl radical which optionally contains one or two hydroxyl groups, or is an N-alkyl radical which optionally contains a hydroxyl group or one or two amine nitrogens, or is an N-pyrrolidinylmethyl or N-piperidinylmethyl or N-morpholinylmethyl radical.

4. Process for preparing the amine according to any of Claims 1 to 3, **characterized in that** 1,2-propylenediamine is reacted
- either, with reductive alkylation, with at least one aldehyde or ketone of the formula (II) and hydrogen 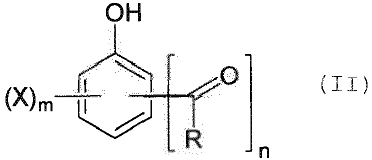
- or, with transamination, with a Mannich base of the formula (III) 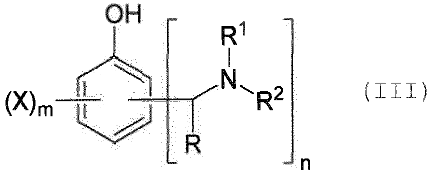 where
R¹ and R² are each identical or different alkyl, cycloalkyl or aralkyl radicals having 1 to 4 carbon atoms and optionally containing ether oxygen or amine nitrogen, or together are an alkylene radical having 4 to 8 carbon atoms and optionally containing ether oxygen or amine nitrogen.

5. Process according to Claim 4, **characterized in that** it is carried out with a ratio between the number of 1,2-propylenediamine molecules and the number of aldehyde or keto groups in the aldehyde or ketone of the formula (II) of at least 1.5/1.

6. Process according to either of Claims 4 and 5, **characterized in that** it is carried out with salicylaldehyde or 2'-hydroxyacetophenone as aldehyde or ketone of the formula (II).

7. Process according to Claim 4, **characterized in that** it is carried out with a ratio between the number of 1,2-propylenediamine molecules and the number of aminoalkyl substituents in the Mannich base of the formula (III) of at least 1/1.

8. Process according to either of Claims 4 and 7, **characterized in that** it is carried out with 2,4,6-tris(N,N-dimethylaminomethyl)phenol as Mannich base of the formula (III).

9. Use of at least one amine according to any of Claims 1 to 3 as hardener for epoxy resins.

10. Hardener for epoxy resins, comprising at least one amine according to any of Claims 1 to 3 and at least one further amine and/or at least one additive.

11. Hardener according to Claim 10, **characterized in that** the further amine is selected from the group consisting of 2-butyl-2-ethyl-1,5-pentanediamine, 2,2(4),4-trimethylhexamethylenediamine, 1,12-dodecandiamine, bis(4-aminocyclohexyl)methane, 1-amino-3-aminomethyl-3,5,5-trimethylcyclohexane, 1,3-bis(aminomethyl)cyclohexane, 1,4-bis(aminomethyl)cyclohexane, 2,5(2,6)-bis(aminomethyl)bicyclo[2.2.1]heptane, 3(4),8(9)-bis(aminomethyl)tricyclo[5.2.1.0^{2,6}]decane, 1,4-diamino-2,2,6-trimethylcyclohexane, 1,8-menthane-diamine, 3,9-bis(3-aminopropyl)-2,4,8,10-tetra-oxaspiro[5.5]undecane, 1,3-bis(aminomethyl)-benzene, aliphatic primary di- and triamines containing ether groups, N¹-benzyl-1,2-propanediamine, N¹-(4-methoxybenzyl)-1,2-propanediamine, N-benzyl-1,3-bis(aminomethyl)benzene, N,N'-dibenzyl-1,3-bis(aminomethyl)benzene, N-2-ethylhexyl-1,3-bis(aminomethyl)benzene, N,N'-bis(2-ethylhexyl)-1,3-bis(aminomethyl)benzene, partially styrenized 1,3-bis(aminomethyl)benzene, and adducts of (i) at least one polyamine having at least three amine hydrogens that are reactive toward epoxide groups with (ii) at least one epoxide.

12. Hardener according to Claim 11, **characterized in that** the further amine is N¹-benzyl-1,2-propanediamine.

13. Epoxy resin composition comprising
- a resin component comprising at least one epoxy resin, and
- a hardener component comprising at least one amine according to any of Claims 1 to 3.

14. Coating obtained from an epoxy resin composition according to Claim 13.

15. Method for accelerating the curing of an epoxy resin composition, **characterized in that** an amine according to any of Claims 1 to 3 is added.

## Revendications

1. Amine de la formule (I) : 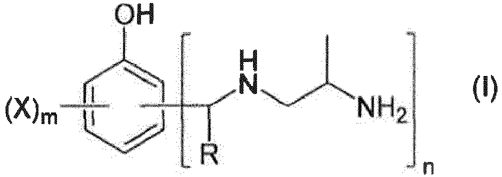 dans laquelle
m représente 0 ou 1 ou 2,
n représente 1 ou 2 ou 3,
R représente un radical hydrogène ou un radical alkyle de 1 à 8 atomes C ou un radical phényle, et
les X représentent des radicaux identiques ou différents choisis dans le groupe constitué par hydroxyle et alkyle, alcényle et alcoxy contenant chacun 1 à 18 atomes C, qui contiennent éventuellement un oxygène d'éther, un oxygène d'hydroxyle ou un azote d'amine, ou représentent un radical N-pyrrolidinylméthyle ou N-pipéridinylméthyle ou N-morpholinylméthyle.

2. Amine selon la revendication 1, **caractérisée en ce que** m représente 0 ou 1, n représente 1, et R représente un radical hydrogène ou un radical méthyle.

3. Amine selon la revendication 1, **caractérisée en ce que** (m+n) représente 3, R représente un radical hydrogène et X représente un radical N,N-dialkyle, qui contient éventuellement un ou deux groupes hydroxyle, ou représente un radical N-alkyle, qui contient éventuellement un groupe hydroxyle ou un ou deux azotes d'amine, ou représente un radical N-pyrrolidinylméthyle ou N-pipéridinylméthyle ou N-morpholinylméthyle.

4. Procédé de fabrication d'une amine selon l'une quelconque des revendications 1 à 3, **caractérisé en ce que** de la 1,2-propylène-diamine est mise en réaction
- soit par alkylation réductrice avec au moins un aldéhyde ou une cétone de la formule (II) et de l'hydrogène 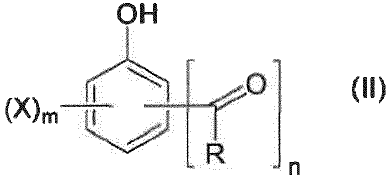
- soit par transamination avec une base de Mannich de la formule (III) 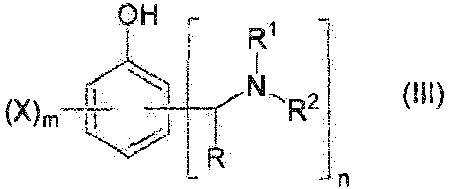 dans laquelle
R¹ et R² représentent chacun des radicaux alkyle, cycloalkyle ou aralkyle identiques ou différents de 1 à 4 atomes C, qui contiennent éventuellement un oxygène d'éther ou un azote d'amine, ou représentent ensemble un radical alkylène de 4 à 8 atomes C, qui contient éventuellement un oxygène d'éther ou un azote d'amine.

5. Procédé selon la revendication 4, **caractérisé en ce qu'**il est réalisé avec un rapport entre le nombre de molécules de 1,2-propylène-diamine et le nombre de groupes aldéhyde ou céto de l'aldéhyde ou de la cétone de la formule (II) d'au moins 1,5/1.

6. Procédé selon l'une quelconque des revendications 4 ou 5, **caractérisé en ce qu'**il est réalisé avec du salicylaldéhyde ou de la 2'-hydroxyacétophénone en tant qu'aldéhyde ou cétone de la formule (II).

7. Procédé selon la revendication 4, **caractérisé en ce qu'**il est réalisé avec un rapport entre le nombre de molécules de 1,2-propylène-diamine et le nombre de substituants aminoalkyle de la base de Mannich de la formule (III) d'au moins 1/1.

8. Procédé selon l'une quelconque des revendications 4 ou 7, **caractérisé en ce qu'**il est réalisé avec du 2,4,6-tris(N,N-diméthylaminométhyl)phénol en tant que base de Mannich de la formule (III).

9. Utilisation d'au moins une amine selon l'une quelconque des revendications 1 à 3 en tant qu'agent de durcissement pour résines époxydes.

10. Agent de durcissement pour résines époxydes, contenant au moins une amine selon l'une quelconque des revendications 1 à 3 et au moins une amine supplémentaire et/ou au moins un additif.

11. Agent de durcissement selon la revendication 10, **caractérisé en ce que** l'amine supplémentaire est choisie dans le groupe constitué par la 2-butyl-2-éthyl-1,5-pentane-diamine, la 2,2(4),4-triméthylhexaméthylène-diamine, la 1,12-dodécane-diamine, le bis(4-aminocyclohexyl)méthane, le 1-amino-3-aminométhyl-3,5,5-triméthylcyclohexane, le 1,3-bis(aminométhyl)cyclohexane, le 1,4-bis(aminométhyl)cyclohexane, le 2,5(2,6)-bis(aminométhyl)bicyclo[2.2.1]heptane, le 3(4),8(9)-bis(aminométhyl)tricycle[5.2.1.0^{2,6}]décane, le 1,4-diamino-2,2,6-triméthylcyclohexane, la 1,8-menthane-diamine, le 3,9-bis(3-aminopropyl)-2,4,8,10-tétraoxa-spiro[5.5]undécane, le 1,3-bis(aminométhyl)benzène, les di- et triamines primaires aliphatiques contenant des groupes éther, la N¹-benzyl-1,2-propane-diamine, la N¹-(4-méthoxybenzyl)-1,2-propane-diamine, le N-benzyl-1,3-bis(aminométhyl)benzène, le N,N'-dibenzyl-1,3-bis(aminométhyl)benzène, le N-2-éthylhexyl-1,3-bis(aminométhyl)benzène, le N,N'-bis(2-éthylhexyl)-1,3-bis(aminométhyl)benzène, le 1,3-bis(aminométhyl)benzène partiellement styrénisé et les adduits de (i) au moins une polyamine contenant au moins trois hydrogènes d'amine réactifs avec les groupes époxyde avec (ii) au moins un époxyde.

12. Agent de durcissement selon la revendication 11, **caractérisé en ce que** l'amine supplémentaire est la N¹-benzyl-1,2-propane-diamine.

13. Composition de résine époxyde, comprenant :
- un composant résine contenant au moins une résine époxyde, et
- un composant agent de durcissement contenant au moins une amine selon l'une quelconque des revendications 1 à 3.

14. Revêtement obtenu à partir d'une composition de résine époxyde selon la revendication 13.

15. Procédé d'accélération du durcissement d'une composition de résine époxyde, **caractérisé en ce qu'**une amine selon l'une quelconque des revendications 1 à 3 est ajoutée.
